Europäisches Patentamt

European Patent Office

Office européen des brevets

(19)

(11) Veröffentlichungsnummer: **0 134 437**
**B1**

(12) **EUROPÄISCHE PATENTSCHRIFT**

(45) Veröffentlichungstag der Patentschrift :
**17.08.88**

(51) Int. Cl.⁴ : **A 61 N 1/42**, A 61 F 13/02,
**H 01 F 1/117**

(21) Anmeldenummer : **84106986.7**

(22) Anmeldetag : **19.06.84**

(54) **Biegsame magnetische Folie.**

(30) Priorität : **29.08.83 DE 3331061**
**14.07.83 DE 3325356**

(43) Veröffentlichungstag der Anmeldung :
**20.03.85 Patentblatt 85/12**

(45) Bekanntmachung des Hinweises auf die Patenterteilung : **17.08.88 Patentblatt 88/33**

(84) Benannte Vertragsstaaten :
**AT BE CH DE FR GB IT LI LU NL SE**

(56) Entgegenhaltungen :
**EP-A- 0 081 109**
**WO-A-84 /003 05**
**AU-A-    424 257**
**DE-A- 1 564 099**
**DE-A- 3 147 852**
**DE-B- 1 943 914**
**FR-A- 2 371 916**

(73) Patentinhaber : **Baermann, Horst**
**Auf dem Saan 36**
**D-5064 Rösrath (DE)**

(72) Erfinder : **Baermann, Horst**
**Auf dem Saan 36**
**D-5064 Rösrath (DE)**

EP 0 134 437 B1

## Beschreibung

Die Erfindung bezieht sich auf eine biegsame magnetische Folie zur Erzeugung eines magnetischen Feldes an Körperteilen, wie im Oberbegriff des Anspruchs 1 und 2 definiert. Es ist bereits eine derartige Folie bekannt, die aus gummiartig-flexiblem Kunststoff besteht, in den dauermagnetische Teilchen aus Ferritwerkstoff, wie z. B. Barium- oder Strontiumferrit, eingebettet sind. Diese Folien sind auf ihrer aktiven, d. h. bei Anwendung dem Körper zugewandten Oberfläche in gleichem Polabstand mit linearen, parallel verlaufenden, streifenförmigen Polen wechselnder Polarität aufmagnetisiert, d. h. einem Nordpolstreifen folgt stets ein Südpolstreifen usw. Zwecks Befestigung an den entsprechenden Körperteilen kann diese Folie auf der magnetisierten Oberfläche mit einer hautverträglichen Selbstklebeschicht versehen sein. (OS-DE-31 47 852).

Dieser magnetischen Folie sind jedoch in ihrer optimalen Anwendung und ihrem Therapieerfolg Grenzen gesetzt.

Man hat festgestellt, daß durch wechselnde Magnetfelder, die auf die zu behandelnden Körperstellen einwirken, geringe elektrische Spannungen bzw. Ströme in der betreffenden Körperstelle induziert werden, durch die eine Erwärmung der Körperteile und eine durchblutungsfördernde Wirkung, insbesondere im periphären Bereich hervorgerufen wird, da sich z. B. die Blutflüssigkeit etwa wie ein elektrisch leitfähiges, bewegtes Medium verhält.

Es wird jedoch eine optimale Therapiewirkung, die sich insbesondere durch Linderung und Behebung von Schmerzen auszeichnet, nur dann erreicht, wenn die Pole abwechselnder Polarität quer, bzw. rechtwinklig zur Flußrichtung des Blutes verlaufen.

Bei der bekannten magnetischen Folie für Therapiezwecke verlaufen die streifenförmigen Pole abwechselnder Polarität nur in einer Richtung, wobei man beim Betrachten der Folie nicht erkennt, in welche Richtung die streifenförmigen Pole verlaufen. Aus diesem Grunde ist es nicht auszuschließen, daß die bekannte magnetische Folie unsachgemäß angebracht wird, so daß die therapeutische Wirkung wenig wirkungsvoll ist. Man weiß somit nicht, ob die Folie so auf der zu behandelnden Körperstelle angebracht ist, daß die therapeutisch optimale Wirkung eintritt. Dieses Problem ist um so größer, da diese magnetischen Folien für therapeutische Zwecke rezeptfrei von jedem Laien, der die eingangs geschilderten Zusammenhänge nicht kennt, überall leicht erworben werden können.

Ein weiterer Nachteil der bekannten Folie besteht darin, daß durch die in einer Richtung verlaufenden, streifenförmigen Pole wechselnder Polarität die Therapiewirkung nur auf die quer dazu verlaufenden Blutgefäße wirkungsvoll ist.

Unter Vermeidung dieser Nachteile, ist es Aufgabe der Erfindung, eine magnetische Folie der eingangs genannten Art zu schaffen, die in jeder beliebigen Lage, ohne besondere Anleitung und, Spezialkenntnisse, auf der Körperstelle angebracht werden kann.

Diese Aufgabe wird erfindungsgemäß durch die Merkmale der Anspruch 1 und 2 gelöst.

In einer vorteilhaften Ausführung ist die magnetische Folie mit Magnetpolen in Form von konzentrisch zueinander angeordneten Ringen versehen, die von Ring zu Ring abwechselnde Polarität besitzen.

Es ist auch eine Ausführung vorteilhaft, bei der auf der magnetischen Folie die Magnetpole in Form von Sektoren vorhanden sind, die von Sektor zu Sektor abwechselnde Polarität aufweisen.

In einer weiteren Ausgestaltung der Erfindung kann man die magnetische Folie so ausbilden, daß dieselbe aus mehreren, mit Polen wechselnder Polarität versehenen Folienteilen besteht, die miteinander, vorzugsweise mittels einer flexiblen unmagnetischen, selbstklebenden Folie verbunden sind.

Durch diese Lösung gelingt es, daß in jeder beliebigen Blutflußrichtung magnetische Pole wechselnder Polarität auf die Blutgefäße einwirken.

Durch die Erfindung wird somit eine magnetische Folie geschaffen, die nicht nur universell, d. h. lageunabhängig anwendbar ist, sondern auf alle Blutflußrichtungen erzielbar ist, insbesondere auch auf das Gefäßsystem im periphären Hautbereich.

Es ist vorteilhaft, wenn die Magnetpole, wie in der Deutschen Patentanmeldung P-33 25 356.0 beschrieben, zueinander eine unterschiedliche Polbreite und/oder einen unterschiedlichen Polabstand besitzen. Bei der sektorförmigen Polausbildung ist dies ohnehin der Fall.

Um die Körperwärme zu halten bzw. zu reflektieren, kann die Folie auf wenigstens einer Oberfläche mit einem metallischen Überzug versehen werden. Dieser Überzug kann z. B. aufgedampft sein.

Zur Vermeidung einer Abnutzung des metallischen Überzuges kann dieser zusätzlich mit einer Schutzschicht, z. B. einem Lack, versehen sein.

Nachfolgend sind vier Ausführungsbeispiele der Erfindung anhand der Zeichnung erläutert.

Es zeigen :

Fig. 1 eine Aufsicht auf die magnetische Folie mit einer ringförmigen Polkonfiguration

Fig. 2 eine Aufsicht auf die magnetische Folie mit sektorförmiger Polkonfiguration

Fig. 3 eine Aufsicht auf die magnetische Folie mit zusammengesetzten Folienteilen

Die biegsame magnetische Folie 1 besteht aus einem hautverträglichen, gummiartig-flexiblen Kunststoff, in den dauermagnetische Teilchen aus Bariumferrit oder Strontiumferrit eingebettet sind.

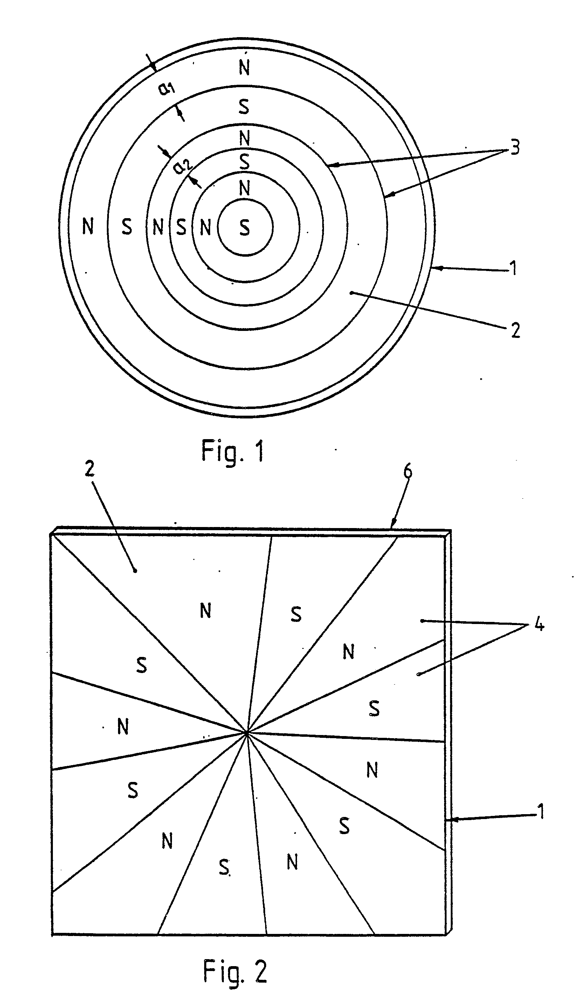

Auf der aktiven Oberfläche, die an der Körperstelle anliegt, ist, wie aus Fig. 1 ersichtlich, die magnetische Folie 1 mit Magnetpolen 2 in Form von konzentrisch zueinander angeordneten Ringen 3 versehen, die von Ring zu Ring abwechselnde Polarität aufweisen. Die Nord- und Südpole sind in der Zeichnung durch die Buchstaben N und S gekennzeichnet. Sie können eine unterschiedliche Polbreite $a_1$, $a_2$ aufweisen.

In einem anderen Ausführungsbeispiel gemäß Fig. 2 ist die magnetische Folie 1 mit Magnetpolen 2 in Form von Sektoren 4 aufmagnetisiert, die von Sektor zu Sektor eine abwechselnde Polarität aufweisen. Auch hier sind die Nord- und Südpole durch die Buchstaben N und S gekennzeichnet.

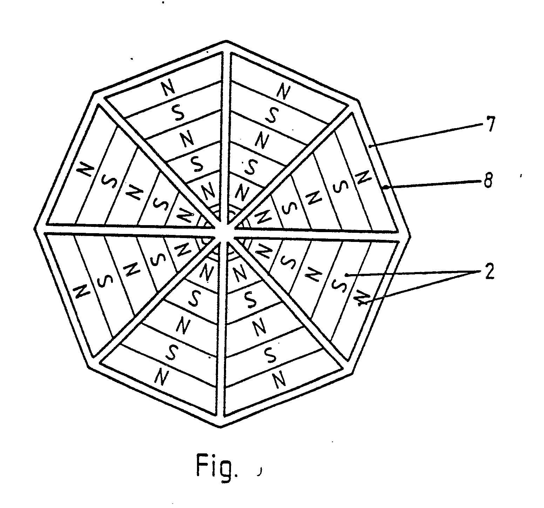

Gemäß einem weiteren Ausführungsbeispiel nach Fig. 3 sind auf einer unmagnetischen, flexiblen Folie 7, die mit einer selbstklebenden Schicht versehen ist, biegsame magnetische Folienteile 8 angebracht, die die Form eines Dreiecks besitzen. Es entsteht somit ein sektorförmiges Gebilde, wobei die Pole 2 wechselnder Polarität zueinander einen winkligen Verlauf aufweisen. Die Nord- und Südpole sind wiederum durch die Buchstaben N und S gekennzeichnet.

Selbstverständlich können die Pole auf der Folie auch jedes andere geometrische Gebilde aufweisen, sofern sie konzentrisch, winklig und/oder radial zueinander derart angeordnet sind, so daß sie senkrecht als auch waagerecht verlaufende Blutflußrichtungen mit ihrer wechselnden Polfolge kreuzen. Die Gestalt der Folie kann hierbei beliebig, z. B. scheibenförmig, rechteckig oder quadratisch, sein.

Zur Befestigung der Folie auf der Haut kann die Folie in bekannter Weise einseitig mit einer hautverträglichen, selbstklebenden Beschichtung — in der Zeichnung nicht dargestellt — versehen sein, die durch eine abziehbare Silikonpapierabdeckung geschützt ist. Die magnetische Folie kann aber auch mit Hilfe von separat beigefügten, hautverträglichen Pflastern befestigt werden.

Um die Körperwärme zu halten bzw. zu reflektieren ist die Folie auf der Rückseite mit einem metallischen Überzug 6 versehen. Der Überzug kann durch Aufkaschieren oder Aufdampfen aufgebracht werden.

Zwecks Vermeidung einer Abnutzung des metallischen Überzuges kann dieser zusätzlich mit einer Schutzschicht, z. B. einem Lack, versehen werden.

**Patentansprüche**

1. Biegsame magnetische Folie zur Erzeugung eines magnetischen Feldes an Körperteilen aus hautverträglichem, gummiartig-flexiblem Kunststoff, in den dauermagnetische Teilchen, vorzugsweise dauermagnetische Ferrite, eingebettet sind, wobei die aktive Fläche der Folie mit Polen abwechselnder Polarität aufmagnetisiert ist, dadurch gekennzeichnet, daß die Magnetpole (2) die Form von ineinanderliegenden Ringen (3) aufweisen, die von Ring zu Ring abwechselnde Polarität besitzen.

2. Biegsame magnetische Folie zur Erzeugung eines magnetischen Feldes an Körperteilen aus hautverträglichem, gummiartig-flexiblem Kunststoff, in den dauermagnetische Teilchen, vorzugsweise dauermagnetische Ferrite, eingebettet sind, wobei die aktive Fläche der Folie mit Polen abwechselnder Polarität aufmagnetisiert ist, dadurch gekennzeichnet, daß die Magnetpole (2) abwechselnder Polarität auf der Folie (1) in radial verlaufenden Flächen angeordnet sind.

3. Biegsame magnetische Folie nach Anspruch 2, dadurch gekennzeichnet, daß die radial verlaufenden Flächen der Magnetpole die Form von Sektoren (4) aufweisen, die von Sektor zu Sektor abwechselnde Polarität besitzen.

4. Biegsame magnetische Folie nach den Ansprüchen 1 oder 2 und 3, dadurch gekennzeichnet, daß dieselbe aus mehreren mit Polen wechselnder Polarität versehenen Folienteilen (8) besteht, die miteinander vorzugsweise mittels einer flexiblen, unmagnetischen, selbstklebenden Folie (7) derart verbunden sind, daß ein zusammenhängendes, aus mehreren Sektoren bestehendes Gebilde vorhanden ist.

**Claims**

1. Flexible magnetic sheet for the purpose of inducing a magnetic field in parts of the body, consisting of a skin-compatible, rubbery flexible synthetic material in which permanent-magnetic particles, preferably permanent-magnetic ferrites, are embedded, the active surface of said sheets having been magnetized with poles of alternating polarity, characterized by the fact that the poles (2) have the shape of rings arranged concentrically to each other which from one ring to the next ring have alternating polarities.

2. Flexible magnetic sheet for the purpose of inducing a magnetic field in parts of the body, consisting of a skin-compatible, rubbery flexible synthetic material in which permanent-magnetic particles, preferably permanent-magnetic ferrites, are embedded, the active surface of said sheets having been magnetized with poles of alternating polarity, characterized by the fact that the poles (2) of alternating polarity are arranged in areas oriented radially.

3. Flexible magnetic sheet according to claim 2, characterized by the fact that the radially oriented areas of the magnetic poles are formed in the shape of sectors (4) the polarities of which alternating from one sector to the next.

4. Flexible magnetic sheet according to claims 1 or 2 and 3, characterized by the fact that the magnetic sheet consists of several sheet elements (8) provided with poles of alternating polarity, said elements being connected to each other, preferably by means of a flexible nonmagnetic self-adhesive sheet (7), so that a joint structure consisting of several sectors is formed.

**Revendications**

1. Feuille flexible magnétique destinée à produire un champ magnétique sur des parties du corps, faite d'une matière plastique flexible de type caoutchouc, non irritante pour la peau, dans laquelle sont incluses des particules magnétiques permanentes, de préférence des ferrites magnétiques permanentes, la surface active de la feuille étant magnétisée avec des pôles de polarité variable, caractérisée en ce que les pôles magnétiques (2) ont la forme d'anneaux concentriques dont la polarité change d'un anneau à l'autre.

2. Feuille flexible magnétique destinée à produire un champ magnétique sur des parties du corps, faite d'une matière plastique flexible de type caoutchouc, non irritante pour la peau, dans laquelle sont incluses des particules magnétiques permanentes, de préférence des ferrites magnétiques permanentes, la surface active de la feuille étant magnétisée avec des pôles de polarité variables, caractérisée en ce que les pôles magnétiques (2) de polarité variable sont disposés sur la feuille (1), sur des surfaces radiales.

3. Feuille flexible magnétique selon la revendication 2, caractérisée en ce que les surfaces radiales des pôles magnétiques ont la forme de secteurs (4) dont la polarité change d'un secteur à l'autre.

4. Feuille flexible magnétique selon les revendications 1 ou 2 et 3, caractérisée en ce qu'elle est constituée de plusieurs parties de feuille (8) pourvues de pôles de polarité variable qui sont assemblées entre elles de préférence au moyen d'une feuille (7) flexible, non magnétique, autocollante, de manière à former une structure continue, constituée de plusieurs secteurs.

Fig. 1

Fig. 2

Fig. ɟ